# EUROPÄISCHE PATENTSCHRIFT

(11) **EP 3 324 862 B1**
(45) Veröffentlichungstag und Bekanntmachung des Hinweises auf die Patenterteilung: **10.03.2021**
(21) Anmeldenummer: 16754408.9
(22) Anmeldetag: 22.07.2016
(51) Int. Cl.: A61B 17/16

(54) **VORRICHTUNG ZUM ABTRENNEN UND ENTFERNEN VON GEWEBETEILEN**
DEVICE FOR SEVERING AND REMOVING TISSUE PARTS
DISPOSITIF DESTINÉ À DÉTACHER ET À EXTRAIRE DES TISSUS

(30) Priorität: 23.07.2015 DE 102015112025; 03.08.2015 DE 102015112716; 28.08.2015 DE 102015114306
(43) Veröffentlichungstag der Anmeldung: 30.05.2018
(73) Patentinhaber: Morpheus AG, 78549 Spaichingen (DE)
(72) Erfinder: RACK, Timo, 78604 Rietheim-Weilheim (DE)
(74) Vertreter: Maucher Jenkins Patent- und Rechtsanwälte
(86) Internationale Anmeldenummer: PCT/EP2016/067497
(87) Internationale Veröffentlichungsnummer: WO 2017/013240

(56) Entgegenhaltungen:
- DE-A1- 3 802 907
- US-A- 4 522 206
- US-A1- 2005 267 503
- US-A1- 2006 122 615
- US-A1- 2011 190 802

## Beschreibung

Die Erfindung betrifft eine Vorrichtung zum Abtrennen und Entfernen von Gewebeteilen insbesondere aus einem Körper eines Lebewesens mittels einer feststehenden Führungsschiene, an der entlang eine Gleitschiene bewegbar ist, wobei Führungsschiene und Gleitschiene im distalen Bereich ein Schneid- oder Stanzmaul zum Abtrennen des Gewebeteils ausbilden.

### STAND DER TECHNIK

Es sind medizinische Vorrichtungen mit einer Schneidzangeneinrichtung zum Entnehmen von Gewebeproben, Entfernen von langgestreckten Körperelementen, wie Nerven- oder Venenabschnitten od.dgl. mit zwei Zangenschenkel bekannt, welche ein Aussenrohr und ein Innenrohr aufweisen. Das Innenrohr dient meist dazu, ein Scherenelement zu betätigen. Durch das Innenrohr wird dann das abgeschnittene Gewebestück abgesaugt.

Sogenannte Gewebe- oder Knochenstanzen sind in vielfältiger Form und Ausführung bekannt und auf dem Markt. Verwiesen wird hier nur beispielsweise auf die DE 195 13 572 C2, in der ein chirurgisches Instrument mit einem Schaft und einem an dem Schaft gleitbar und lösbar angeordneten Schlitten aufgezeigt ist. Der Schlitten ist dabei lösbar mit einem Griffteil und der Schaft mit einem Hauptgriffteil verbunden. Am distalen Ende des Schaftes ragt ein Gegenelement auf, gegen das eine Schneidkante am distalen Ende des Schlittens gefahren werden kann. Hierdurch wird ein Gewebeteil ausgestanzt.

Aus der US 2006/0122615 A1 und der US 5 653 713 A sind Gewebestanzen bekannt, bei denen ein Kanal ausgebildet wird, der zum Absaugen von Gewebeteilen dient.

US 2011/0190802 A2 offenbart eine Vorrichtung zum Abtrennen und Entfernen von Gewebeteilen mittels einer feststehenden Führungsschiene, an der entlang eine Gleitschiene bewegbar ist, wobei die Gleitschiene einen Absaugkanal zum Entfernen des abgetrennten Gewebeteils ausbildet, wobei Führungsschiene und Gleitschiene im distalen Bereich ein Schneid- oder Stanzmaul zum Abtrennen des Gewebeteils ausbilden, wobei dem Schneid- oder Stanzmaul eine Leitung zum Zuführen einer Flüssigkeit zugeordnet ist, wobei am distalen Ende der Führungsschiene ein Schneidgegenelement aufragt.

US 2005/0267503 A1 offenbart ähnliche Vorrichtungen, wobei bei einem Ausführungsbeispiel die beiden Schneidkanten des Schneid- oder Stanzmauls schräg zueinander angestellt sind.

### AUFGABE

Aufgabe der vorliegenden Erfindung ist es, eine derartig bekannte Knochen- oder Gewebestanze vielfältiger verwendbar auszugestalten und die Absaugung zu verbessern.

### LÖSUNG DER AUFGABE

Zur Lösung der Aufgabe führen die Merkmale nach dem Anspruch 1.

Diese Flüssigkeit dient nicht nur dazu, den Operationsraum zu spülen, sondern soll im Wesentlichen zusammen mit dem zu entfernenden Gewebeteil abgesaugt werden. Das bedeutet, dass zwischen Absaugkanal und Austrittsöffnung der Leitung quasi ein Kurzschluss gebildet wird, so dass die Flüssigkeit gleich nach dem Austreten aus der Leitung in den Absaugkanal eintritt. Dies gewährleistet, dass die Gewebeteile durch den Absaugkanal gespült werden.

Unter Gewebeteilen werden insbesondere, aber nicht abschliessend, Muskelgewebe, Binde- und Stützgewebe, einschliesslich Knochenteile, Knorpel und Fettgewebe, Epithelgewebe und auch Nervengewebe verstanden. Es soll jedes Teil eines Lebewesens umfassen, das mit einer üblichen Gewebe- bzw. Knochenstanze entfernt und entnommen werden kann.

Bei den bisher bekannten Gewebe- bzw. Knochenstanzen sind Gleitschiene und Führungsschiene in der Regel über beinahe ihre gesamte Länge in Abständen miteinander verbunden. Meist ragt von einem Teil ein Nutenstein ab, der in einer T-Nut in dem anderen Teil geführt wird. Dies ist natürlich auch bei der vorliegenden Erfindung möglich, bevorzugt wird allerdings, dass Gleitschiene und Führungsschiene nur am distalen Ende miteinander gekoppelt sind. Hier ist jede Art von Führung denkbar und soll von der Erfindung umfasst sein. In einem einfachen Ausführungsbeispiel weist die Führungsschiene am distalen Ende bevorzugt auf beiden Seiten eine Führungsnut auf, in die jeweils eine Schiene der Gleitschiene eingreift.

Um eine eigentliche Stanze auszubilden, ist am distalen Ende der Führungsschiene ein aufragendes Schneidgegenelement vorgesehen. Dabei ist es am besten, wenn dieses Schneidgegenelement einstückig aus der Führungsschiene herausgeformt ist.

Das Schneidgegenelement bildet an seinem Rand eine halbkreisförmige Schneidkante aus, die mit einer halbovalen Schneidöffnung in dem distalen Ende der Gleitschiene zusammenwirkt. Erfindungsgemäß kann das Schneidgegenelement in die Schneidöffnung der Schneidschiene einfahren. D.h., beim Bewegen der Gleitschiene entlang der Führungsschiene wird das distale Ende der Gleitschiene über das Schneidgegenelement geführt, wobei die beiden Schneidkanten scherenartig zusammenwirken. Dabei ist vorgesehen, die eine Schneidkante gegenüber der anderen Schneidkante schräg anzustellen, so dass sich das Schneid- und Stanzmaul scherenartig schliesst und so der Schneidvorgang wesentlich verbessert wird.

Einem besseren Abführen der Gewebeteile dient auch, wenn sich der von dem Schneid- und Stanzmaul gebildete Hohlraum zum Absaugkanal hin öffnet. Dies kann auch stufenförmig geschehen.

Der Absaugkanal beginnt mit dem Schneid- oder Stanzmaul beziehungsweise einem von diesem Maul gebildeten Auffangraum für Gewebeteile. Anderenends geht er in einen Anschlussnippel für beispielsweise einen Absaugschlauch über, so dass dann das Gewebeteil beispielsweise in einem Behälter gefangen werden kann. Bevorzugt ist mit diesem Anschlussnippel noch ein Ventil vorgeschaltet, mit dem es möglich ist, den Absaugkanal auch abzusperren, so dass die Vorrichtung ganz allgemein als Schneid- oder Knochenstanze verwendet werden kann.

Die Gleitschiene soll mit der Führungsschiene verbunden sein. Erfindungsgemäß wird die Führungsschiene feststehend ausgebildet und die Gleitschiene entlang dieser feststehenden Führungsschiene verschiebbar sein. Wie die Verbindung erfolgt, ist von untergeordneter Bedeutung. Gedacht ist beispielsweise an eine Führung von Nutensteinen an der einen Schiene in entsprechenden Nuten der anderen Schiene. Sollte die Vorrichtung auch leicht lösbar beziehungsweise zerlegbar sein, so sind diese Verbindungen entsprechend ausgestaltet. Hierfür gibt es genügend Beispiele im Stand der Technik.

Ein wesentliches Element der vorliegenden Erfindung ist die Ausgestaltung des Schneid- oder Stanzmauls. Zum einen ist daran gedacht, ein Teil dieses Schneid-oder Stanzmauls feststehend auszugestalten. Dabei kann dieser Teil zum Beispiel einstückig aus der Führungsschiene herausgeformt sein. Denkbar ist aber auch die Anordnung an einem Aufschub, welcher, bevorzug lösbar, mit der Führungsschiene verbunden wird. Die Lösbarkeit gewährleistet, dass der feststehende Teil durch einen beliebigen anderen, auch bewegbaren Teil ausgetauscht werden kann, ohne dass es wesentlicher Veränderungen der gesamten Vorrichtung bedarf.

Gemäß der Erfindung soll dem Schneid- oder Stanzmaul Flüssigkeit zugeführt werden können. Dabei kann es sich beispielsweise um Wasser handeln. Dies geschieht über eine Leitung, die nahe dem Schneid- oder Stanzmaul austritt.

### FIGURENBESCHREIBUNG

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- **Figur 1**: eine Draufsicht auf eine erfindungsgemässe Vorrichtung zum Abtrennen und Entfernen von Gewebeteilen mit einem geöffneten Schneid- oder Stanzmaul;
- **Figur 2**: eine vergrössert dargestellte Draufsicht auf ein distales Ende der erfindungsgemässen Vorrichtung gemäss Figur 1 mit einem teilweise geschlossenen Schneid- oder Stanzmaul;
- **Figur 3**: eine vergrössert dargestellte Draufsicht auf den distalen Bereich der erfindungsgemässen Vorrichtung gemäss Figur 2 mit vollkommen geschlossenem Schneid- oder Stanzmauls;
- **Figur 4**: eine perspektivische Ansicht des distalen Bereichs der erfindungsgemässen Vorrichtung gemäss Figur 1, wobei ein Teilbereich eines distalen Endes einer Gleitschiene durchscheinend ausgebildet ist.

Eine erfindungsgemässe Vorrichtung P zum Abtrennen und Entfernen von Gewebeteilen 20, insbesondere aus einem Körper eines Lebewesens, ist ähnlich einer bekannten Gewebestanze ausgebildet. Die Vorrichtung P weist eine Führungsschiene 1 auf, die in diesem Ausführungsbeispiel einstückig in einen Zangengriff 2 übergeht. Mit dem Zangengriff 2 ist über ein Drehgelenk 3 ein weiterer Zangengriff 4 verbunden, der eine gelenkige Verbindung 5 mit einer Gleitschiene 6 eingeht. Nach der gelenkigen Verbindung 5 mündet die Gleitschiene 6 in ein Drehventil 7 ein, das in einen Anschlussnippel 8, beispielsweise zum Anschliessen eines Absaugschlauches, übergeht. Durch eine Schliessbewegung der beiden Zangengriffe 2 und 4 gegeneinander erfolgt ein Bewegen der Gleitschiene 6 gegenüber der Führungsschiene 1, wobei die Gleitschiene 6 am distalen Ende der Vorrichtung P mit der Führungsschiene 1 verbunden ist. Zu diesem Zweck sind beidseits in der Führungsschiene 1 Führungsnuten 9 ausgebildet, in denen jeweils eine nach innen gerichtete Schiene 10 der Gleitschiene 6 geführt ist.

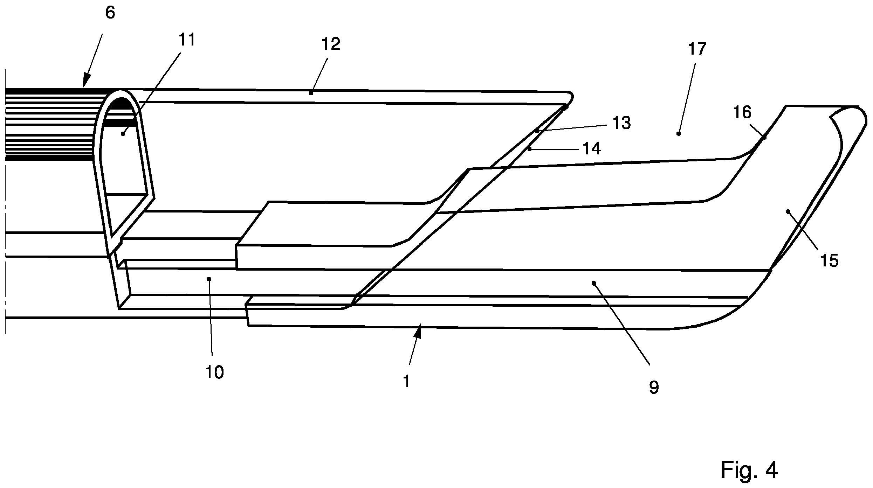

Erfindungsgemäss ist die Gleitschiene 6, wie insbesondere in Figur 4 erkennbar, hohl ausgebildet und gestaltet so einen Absaugkanal 11. Dieser Absaugkanal 11 mündet am distalen Ende der Gleitschiene 6 in ein Schneidstück 12 aus, welches eine Schneidöffnung 13 ausbildet. Diese Schneidöffnung 13 wird von einer Schneidkante 14 umrandet. Die Schneidöffnung 13 ist dabei halb oval ausgebildet.

Da das Schneidstück 12 nach unten hin offen ist, umfasst es einen distalen Bereich der Führungsschiene 1 beidseitig und bildet dort die oben erwähnten Schienen 10, die in den Führungsnuten 9 der Führungsschiene gleiten.

Der Führungsschiene 1 ist am distalen Ende ein Schneidgegenelement 15, im gezeigten Ausführungsbeispiel einstückig, angeformt. Zur Schneidöffnung 13 der Gleitschiene 6 hin bildet das Schneidgegenelement 15 eine halbkreisförmige Schneidkante 16 aus, die in ihrer Kontur der Schneidöffnung 13 angepasst ist. Schneidöffnung 13 und Schneidgegenelement 15 mit der Schneidkante 16 sind so ausgestaltet, dass das Schneidgegenelement 15 in der Schneidöffnung 13 aufgenommen werden kann, wenn die Gleitschiene 6 in Schliesslage bewegt wird, wobei sich das von den beiden Schneidkanten 14 und 16 gebildete Schneid- bzw. Stanzmaul 17 schliesst.

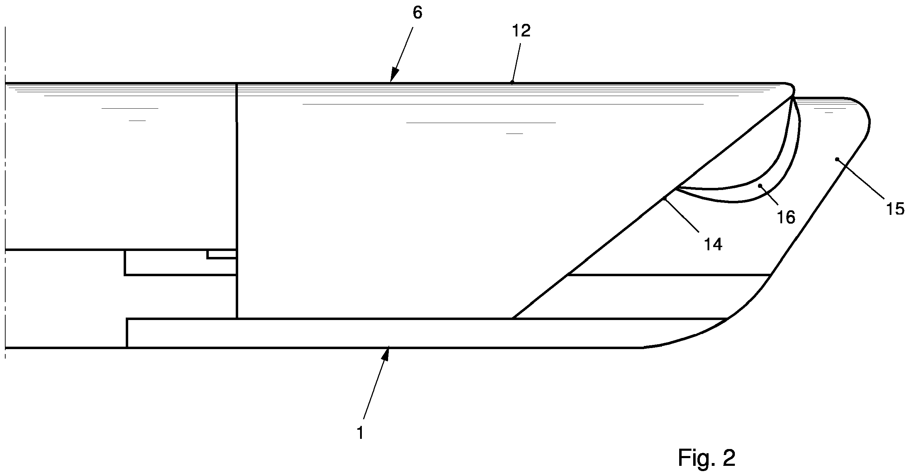

Wie insbesondere in Figur 2 erkennbar, ist die Schneidkante 14 schräg gegenüber der Schneidkante 16 angestellt, sodass beim Schliessen des Schneid- oder Stanzmaules 17 zwischen beiden Schneidkanten 14 und 16 eine scherenartige Schliessbewegung stattfindet, durch die die Schneidwirkung wesentlich verbessert wird.

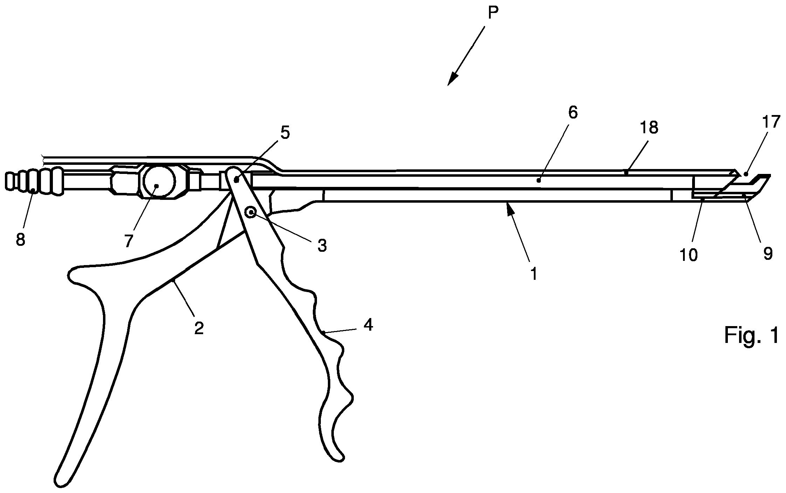

In Figur 1 ist zudem angedeutet, dass sich auf der Gleitschiene 6 eine Leitung 18 zum Zuführen einer Flüssigkeit zu dem Schneid- oder Stanzmaul 17 befindet. Diese Leitung 18 hat einen Anschluss an eine nicht näher gezeigte Flüssigkeitsquelle.

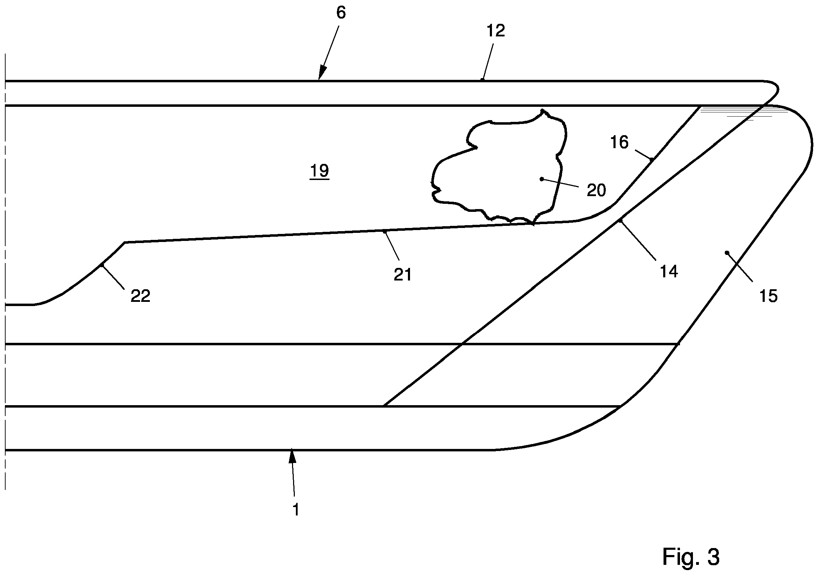

Die Funktionsweise der vorliegenden Erfindung ist folgende:
Die erfindungsgemässe Vorrichtung kann beispielsweise durch einen Trokar oder eine entsprechend erzeugte Körperöffnung in den Körper eines Menschen eingeführt werden, um ein Gewebeteil 20 gleich welcher Art zu entnehmen. Dieses Gewebeteil 20 wird in den Bereich des Schneid- oder Stanzmauls 17 gebracht, sodann wird das Schneid- oder Stanzmaul 17 geschlossen. Hierbei wird das Gewebe von den beiden Schneidkanten 14 und 16 abgeschert und in einem, insbesondere in Figur 3 gezeigten Hohlraum 19 aufgenommen. Dabei ist erkennbar, dass ein Boden 21 des Hohlraums 19, welcher durch die Führungsschiene 1 ausgebildet wird, nach hinten leicht abfällt, sodass sich der Hohlraum 19 nach hinten zum Absaugkanal 11 hin öffnet. Dies erleichtert das Absaugen des Gewebeteils 20. Nochmals erleichtert wird dies durch eine Stufe 22 kurz vor dem Absaugkanal 11. Wird das Schneidstück 12 beim Öffnen des Schneid- oder Stanzmauls nach hinten bewegt, nimmt es das Gewebeteil 20 bereits mit, sodass die Absaugwirkung in dem Absaugkanal 11 unterstützt wird.

Eine weitere Unterstützung der Absaugung des Gewebeteils 20 findet durch die zugeführte Flüssigkeit aus der Leitung 18 statt. Insbesondere wenn sich das Schneid- oder Stanzmaul 17 schliesst, erhöht sich die Saugwirkung in diesem Bereich düsenartig, sodass auch ein direktes Ansaugen der Flüssigkeit durch das Schneid- oder Stanzmaul 17 direkt in den Absaugkanal 11 stattfindet, wodurch ein gewisses Spülen des Absaugkanals 11 durchgeführt werden kann, sodass auch fest steckende Gewebeteile 20 mitgeschwemmt werden. Zu diesem Zweck mündet die Leitung 18 bevorzugt kurz oberhalb der Schneidöffnung 13 aus.

**Bezugszeichenliste**

| | | | | | |
|---|---|---|---|---|---|
| 1 | Führungsschiene | | | | |
| 2 | Zangengriff | | | | |
| 3 | Drehgelenk | | | | |
| 4 | Zangengriff | | | | |
| 5 | gelenkige Verbindung | | | | |
| 6 | Gleitschiene | | | | |
| 7 | Ventil | | | | |
| 8 | Anschlussnippel | | | | |
| 9 | Führungsnut | | | | |
| 10 | Schiene | | | | |
| 11 | Absaugkanal | | | | |
| 12 | Schneidstück | | | | |
| 13 | Schneidöffnung | | | | |
| 14 | Schneid kante | | | | |
| 15 | Schneidgegenelement | | | | |
| 16 | Schneid kante | | | | |
| 17 | Schneid- bzw. Stanzmaul | | | | |
| 18 | Leitung | | | | |
| 19 | Hohlraum | | | | |
| 20 | Gewebeteile | | | | |
| 21 | Boden | | | | |
| 22 | Stufe | | | | |
| | | | | | |
| | | | | | |
| | | | | | |
| | | | | | |
| | | | | P | Vorrichtung |
| | | | | | |
| | | | | | |
| | | | | | |
| | | | | | |
| | | | | | |

## Patentansprüche

1. Vorrichtung zum Abtrennen und Entfernen von Gewebeteilen (20), insbesondere aus einem Körper eines Lebewesens , mittels einer feststehenden Führungsschiene (1), an der entlang eine Gleitschiene (6 ) bewegbar ist, wobei die Gleitschiene (6) einen Absaugkanal (11) zum Entfernen des abgetrennten Gewebeteils (20) ausbildet, wobei Führungsschiene (1,30) und Gleitschiene (6 ) im distalen Bereich ein Schneid- oder Stanzmaul (17) zum Abtrennen des Gewebeteils (20) ausbilden, wobei dem Schneid- oder Stanzmaul (17) eine Leitung (18) zum Zuführen einer Flüssigkeit zugeordnet ist, wobei am distalen Ende der Führungsschiene (1) ein Schneidgegenelement (15) aufragt, wobei das Schneidgegenelement (15) eine halbkreisförmige Schneidkante (16) ausbildet, wobei die Gleitschiene (6) an ihrem distalen Ende eine halbovale Schneidöffnung (13) ausbildet, wobei eine Schneidkante (14) der Schneidöffnung (13) der Gleitschiene (6 ) schräg zu der Schneidkante (16) des Schneidgegenelementes (15) angestellt ist, wobei ein Winkel zwischen einer Längsachse der Gleitschiene (6 ) und der Schneidkante (14) der Schneidöffnung (13) der Gleitschiene ( 6) kleiner als ein Winkel zwischen einer Längsachse der Führungsschiene (1) und der Schneidkante (16) des Schneidgegenelementes (15) ist, wobei beim Bewegen der Gleitschiene entlang der Führungsschiene ein distales Ende der Gleitschiene (6 ) über das Schneidgegenelement (15) führbar ist, so dass die beiden Schneidkanten (14,16) scherenartig zusammenwirken.

2. Vorrichtung nach Anspruch 1, **dadurch gekennzeichnet, dass** die Leitung (18) oberhalb der Schneidkante (14) der Schneidöffnung (13) der Gleitschiene (6) ausmündet.

3. Vorrichtung nach wenigstens einem der vorigen Ansprüche, **dadurch gekennzeichnet, dass** eine Kontur der Schneidöffnung (13) am distalen Ende der Gleitschiene (6) mit einer Kontur der halbkreisförmigen Schneidkante (16) am Schneidgegenelement (15) zusammenwirkt, wobei das Schneidgegenelement (15) in die Schneidöffnung (13) einfährt.

4. Vorrichtung nach wenigstens einem der vorhergehenden Ansprüche, **dadurch gekennzeichnet, dass** die Führungsschiene (1) im Bereich des Schneid- oder Stanzmauls (17) eine Stufe (22) ausbildet.

## Claims

1. Device for severing and removing tissue parts (20), in particular from a body of a living being, by means of a fixed guide rail (1) along which a slide rail (6) is movable, wherein the slide rail (6) forms a suction channel (11) for removal of the severed tissue part (20), wherein guide rail (1) and slide rail (6) form, in the distal region, a cutting or punching mouth (17) for severing the tissue part (20), wherein a conduit (18) for supplying a liquid is assigned to the cutting or punching mouth (17), wherein a mating cutting element (15) protrudes at the distal end of the guide rail (1), wherein the mating cutting element (15) forms a semicircular cutting edge (16), wherein the slide rail (6), at its distal end, forms a semi-oval cutting opening (13), wherein a cutting edge (14) of the cutting opening (13) of the slide rail (6) is set at an incline to the cutting edge (16) of the mating cutting element (15), wherein an angle between a longitudinal axis of the slide rail (6) and the cutting edge (14) of the cutting opening (13) of the slide rail (6) is smaller than an angle between a longitudinal axis of the guide rail (1) and the cutting edge (16) of the mating cutting element (15), wherein, during the movement of the slide rail along the guide rail, a distal end of the slide rail (6) can be guided over the mating cutting element (15), such that the two cutting edges (14, 16) interact like scissors.

2. Device according to Claim 1, **characterized in that** the conduit (18) opens out above the cutting edge (14) of the cutting opening (13) of the slide rail (6).

3. Device according to at least one of the preceding claims, **characterized in that** a contour of the cutting opening (13) at the distal end of the slide rail (6) interacts with a contour of the semicircular cutting edge (16) at the mating cutting element (15), wherein the mating cutting element (15) travels into the cutting opening (13).

4. Device according to at least one of the preceding claims, **characterized in that** the guide rail (1) forms a step (22) in the region of the cutting or punching mouth (17) .

## Revendications

1. Dispositif destiné à détacher et à extraire des tissus (20), en particulier du corps d'un être vivant, au moyen d'un rail de guidage fixe (1) le long duquel un rail coulissant (6) peut se déplacer, dans lequel le rail coulissant (6) forme un canal d'aspiration (11) destiné à extraire les tissus (20) détachés, dans lequel le rail de guidage (1) et le rail coulissant (6) constituent dans la zone distale une mâchoire à découper ou à estamper (17) destinée à détacher les tissus, dans lequel une conduite (18) destinée à introduire un liquide est associée à la mâchoire à découper ou à estamper (17), dans lequel un élément de contre-lame (15) fait saillie à l'extrémité distale du rail de guidage (1), dans lequel l'élément de contre-lame (15) forme une arête coupante semi-circulaire (16), dans lequel le rail coulissant (6) forme à son extrémité distale un orifice de coupe semi-ovale (13), dans lequel une arête coupante (14) de l'orifice de coupe (13) du rail coulissant (6) est disposée obliquement par rapport à l'arête coupante (16) de l'élément de contre-lame (15), dans lequel un angle entre un axe longitudinal du rail coulissant (6) et l'arête coupante (14) de l'orifice de coupe (13) du rail coulissant (6) est plus petit qu'un angle entre un axe longitudinal du rail de guidage (1) et l'arête coupante (16) de l'élément de contre-lame (15), dans lequel, lors du déplacement du rail coulissant le long du rail de guidage, une extrémité distale du rail coulissant (6) peut être guidée au-dessus de l'élément de contre-lame (15), de sorte que les deux arêtes coupantes (14, 16) coopèrent à la façon de ciseaux.

2. Dispositif selon la revendication 1, **caractérisé en ce que** la conduite (18) débouche au-dessus de l'arête coupante (14) de l'orifice de coupe (13) du rail coulissant (6).

3. Dispositif selon au moins une des revendications précédentes, **caractérisé en ce qu'**un contour de l'orifice de coupe (13) à l'extrémité distale du rail coulissant (6) coopère avec un contour de l'arête coupante semi-circulaire (16) de l'élément de contre-lame (15), l'élément de contre-lame (15) pénétrant dans l'orifice de coupe (13).

4. Dispositif selon au moins une des revendications précédentes, **caractérisé en ce que** le rail de guidage (1) forme un épaulement (22) dans la zone de la mâchoire à découper ou à estamper (17).
